(19) Europäisches Patentamt
European Patent Office
Office européen des brevets

(11) Publication number: **0 244 027 B1**

# (12) EUROPEAN PATENT SPECIFICATION

(45) Date of publication of patent specification: **10.07.91**

(51) Int. Cl.$^5$: **C07D 211/46**

(21) Application number: **87200772.9**

(22) Date of filing: **23.04.87**

(54) **Process for the synthesis of allylated derivatives of 2,2,6,6-tetraalkylpiperidinols.**

(30) Priority: **02.05.86 IT 2029686**

(43) Date of publication of application:
**04.11.87 Bulletin 87/45**

(45) Publication of the grant of the patent:
**10.07.91 Bulletin 91/28**

(84) Designated Contracting States:
**AT BE CH DE ES FR GB GR LI LU NL SE**

(56) References cited:
**EP-A- 0 001 803**
**EP-A- 0 015 237**
**EP-A- 0 158 598**
**EP-A- 0 162 524**
**DE-A- 2 040 983**
**DE-A- 2 258 752**

**CHEMICAL ABSTRACTS, vol. 100, no. 12, 19th March 1984, page 37, abstract no. 86677u, Columbus, Ohio, US; & JP-A-58 108 238**

(73) Proprietor: **ENICHEM SYNTHESIS S.p.A.**
**Via Ruggero Settimo 55**
**I-90139 Palermo(IT)**

(72) Inventor: **Costanzi, Silvestro**
**Via Dei Mille 26**
**I-20098 San Giuliano Milanese Milan(IT)**
Inventor: **Pallini, Luciano**
**Via V. Bottego 3**
**I-43045 Fornovo Taro Parma(IT)**
Inventor: **Gussoni, Damiano**
**Via Inama 7**
**I-20133 Milan(IT)**

(74) Representative: **Roggero, Sergio et al**
**Ing. Barzanò & Zanardo Milano S.p.A. Via Borgonuovo 10**
**I-20121 Milano(IT)**

**EP 0 244 027 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a process for the synthesis of a 4-allyloxy-2,2,6,6-tetraalkylpiperidinic derivative having the formula (I):

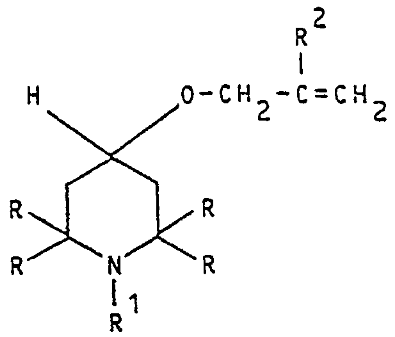

(I)

wherein the four substituents R are alkyl groups containing from 1 to 4 carbon atoms, $R^1$ is a hydrogen atom, an alkyl group containing from 1 to 20 carbon atoms, an alkenyl group containing from 3 to 20 carbon atoms, a phenylalkyl group containing from 7 to 12 carbon atoms, or a group having the formula

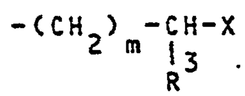

wherein m is 0, 1, 2, or 3, $R^3$ is hydrogen, methyl or phenyl and X represents a halogen atom or a cyano, $-COR^4$, $-COOR^4$, $-COSR^4$, $-CONR^4R^5$, or $-CSNR^4R^5$, group, wherein $R^4$ is an alkyl group containing from 1 to 4 carbon atoms, and $R^5$ is a hydrogen atom or an alkyl group containing from 1 to 4 carbon atoms, and $R^2$ is either hydrogen or a methyl group, by means of the allylation of the corresponding piperidinol having the formula (II):

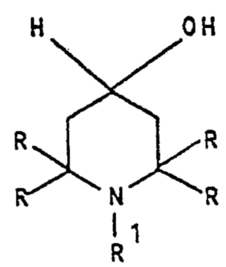

(II)

wherein R and $R^1$ have the above-defined meanings, with an allyl halide of formula (III)

$CH_2=CR^2-CH_2Y$ (III)

wherein $R^2$ is as defined above, and Y is a chlorine or bromine atoms, characterized in that the piperidinic derivative of formula (II) is contacted with an at least equimolar amount of the allyl halide (III) in the presence of an at least equimolar amount of a finely subdivided alkaline hydroxide and of catalytic amounts of a phase-transfer catalyst, in the absence of solvents, or in the presence of an inert organic solvent.

N-substituted 2,2,6,6-tetramethylpiperidinic derivatives bearing an alkenyloxy group in the 4-position, as well as their use as stabilizers for synthetic polymers are claimed in the German patent application No. 2,258,752.

Compounds having the formula (I) wherein $R^1$ is a hydrogen atom are instead useful as intermediates for the stabilizer compounds of the above mentioned German patent application No. 2,258,752, as well as for further stabilizers, such as, e.g., those as disclosed in **European** patent application **Publication Nr. 0162524 on 27.11.1985.**

In the German patent application No. 2,258,752 the preparation is disclosed of 4-allyloxy-1-allyl-2,2,6,6-tetramethylpiperidine by means of the N-allylation with allyl bromide of 4-allyloxy-2,2,6,6-tetramethylpiperidine. The reaction leads, besides to the desired product, to the formation of 4-allyloxy-2,2,6,6-tetramethylpiperidine hydrobromide too, wherein the starting compound acts as the acceptor of the hydrobromic acid which is formed during the reaction, the yields thereof being thus halved.

The preparation of 4-allyloxy-2,2,6,6-tetramethylpiperidine by 0-allylation of 2,2,6,6-tetramethylpiperidinol is disclosed on the contrary in **European** patent application **Pub. Nr.0162524** and, in particular, in Example 1 thereof. In such Example, the alcoholic hydroxyl of piperidine is etherified with allyl halides according to the so-called Williamson's synthesis, which precisely provides the synthesis of ethers by passing through the intermediate formation of the corresponding alkoxides.

But such a synthesis apperas to be not convenient from an industrial standpoint, in as much as for it a two-step process must be in fact provided, wherein the alkoxide intermediate is first formed, and is subsequently converted into the allyl ether by the addition of allyl halides. Furthermore, as reported in the cited patent application, the yields supplied by this reaction are lower than 70%.

The application is known as well to the Williamson's synthesis of the phase-transfer technique, according to which the halide is reacted with the alcohol in 50% aqueous solution of KOH or NaOH, and in the presence of a phase transfer catalyst, thus the desired unsymmetrical ether being obtained. In this particular case, such a reaction suffers anyway from the disadvantage that it also leads to the formation of allyl alcohol, and, hence, of diallyl ether, thus the reaction yields, as computed relatively to the allyl halide, being reduced under 70%.

Is has been surprisingly found now that by reacting a piperidinic derivative having the formula (II):

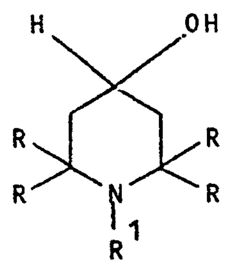

(II)

wherein R and $R^1$ have the above-defined meanings, with an at least equimolar amount of a allyl halide of formula (III)

$CH_2=CR^2\text{-}CH_2Y$ (III)

wherein $R^2$ is hydrogen or methyl, and Y is a chlorine or bromine atom, in the presence of an at least equimolar amount of a finely subdivided alkaline hydroxide and of catalytic amounts of a phase-transfer catalyst, in the absence of solvents, or in the presence of an inert organic solvent, the desired unsymmetrical ether of formula (I) is obtained with yields close to, or higher than, 90%, relatively to both of the starting products.

Although the reaction proceeds well even when equimolar amounts are used of the allyl halide of formula (III), in general the use of a large excess of such a reactant is preferred. This allows indeed the use of a further solvent to be avoided, without other types of problems being generated, in that unreacted allyl halide can be easily distilled off at reaction end, and recycled.

According to a preferred practical embodiment of the present invention, the reaction is carried out by using an excess of the allyl halide (III) comprised within the range of from 100 to 400% by mol, relatively to the stoichiometric amount. Also as regards the alkaline hydroxide, even if an equimolar amount thereof according to the reaction stoichiometry, is enough, better results are obtained by using an excess thereof.

Optimum results were obtained in particular by using the alkaline hydroxide in an excess comprised within the range of from 30 to 150% by mol, relatively to the stoichiometric amount. Preferred alkaline hydroxides will be, due to obvious reasons of availability and cheapness, sodium hydroxide and potassium hydroxide.

The reaction requires also the presence of catalytic amounts of a phase-transfer catalyst, which can be selected from the quaternary ammonium salts and the crown ethers.

The obtainment of the best results, both in terms of yields and of lower cost, make the quaternary ammonium hydroxides be preferrred; among them, in particular tetrabutylammonium iodide, bromide, chloride and sulphate, benzyltriethylammonium chloride, tetrapropylammonium bromide and trimethylhexadecylammonium fluoride have shown to be particularly satisfactory.

The phase-transfer catalyst is generally used at percentages comprised within the range of from 0.005 to 2%, and preferably of from 0.1% to 0.5% by mol, relatively to the piperidinic substrate of formula (II) used as the starting product.

As already seen, the reaction can be carried out in the absence of solvents, in particular when a large excess of the allyl halide is used, or in the presence of a suitable polar, aprotic organic solvent which does not interfere negatively during the reaction course.

Suitable organic solvents are, e.g., the alkyl ethers, such as methyl-tert-butyl ether, diisopropyl ether, diisobutyl ether, dimethoxyethane, and so forth; the cyclic ethers, such as dioxane and tetrahydrofuran, and still further analogous solvents.

The O-allylation reaction of the present invention is generally carried out at a temperature comprised within the range of from 20 °C to 120 °C. Although the reaction may proceed also at room temperature, in general it is preferred to accelerate the rate thereof by operating at the reaction mixture reflux temperature. In general, the reaction is complete, according to the temperature it is carried out at, within a time range of from 1 to 24 hours. At reaction end, the desired product of formula (I) is recovered, by using conventional techniques which will be immediately evident to anyone skilled in the chemical art.

A convenient method provides, e.g., the addition, to the reaction mixture, of water, and of an organic solvent immiscible with water,, e.g., benzene, toluene, xylene, anisole, heptane, cyclohexane, etc., the separation of the organic phase, and the removal from this of the solvent and of the unreacted allyl halide, followed, if desired, by the possible purification of the so-obtained residue, by distillation under reduced pressure.

The compounds obtained by means of the process of the present invention are useful as stabilizers for polymers, or as intermediates for the preparation of stabilizers for polymers.

On considering that, a preferred form of practical embodiment of the present invention provides the use of the new process for the preparation of piperidinic derivatives of formula (I), wherein the four R groups represent a methyl group, $R^1$ is a hydrogen atoms, an alkyl group containing from 1 to 20 carbon atoms, an alkenyl group containing from 3 to 20 carbon atoms, or a phenylalkyl group containing from 7 to 12 carbon atoms, and $R^2$ is either hydrogen or methyl.

To the purposes of the present Applicant, a more preferred practical embodiment of the present invention is represented by the use of the new process for the preparation of piperidinic derivatives of formula (I), wherein the four R substituents are methyl, $R^1$ is a hydrogen atom, an alkyl group containing from 1 to 20 carbon atoms or an alkenyl group containing from 3 to 20 carbon atoms, and $R^2$ is either hydrogen or methyl.

The following Examples disclose in greater detail some representative aspects of the process of the present invention.

Example 1

Preparation of 4-allyloxy-2,2,6,6-tetramethylpiperidine

To a 3-neck 500-ml flask, equipped with mechanical stirrer, reflux condenser and thermometer, 2,2,6,6-tetramethyl-piperidin-4-ol(62 g; 0.395 mol), NaOH powder (31 g; 0.775 mol), allyl chloride (100 g, 1.317 mol) and tetrabutylammonium iodide (0.4 g; 0.001 mol) are charged. The mixture, strongly stirred, is kept heated at reflux temperature about 6 hours. It is then cooled to room temperature, and to it $H_2O$ (150 ml) and toluene (115 ml) are added.

The organic phase is separated and submitted to distillation under atmospheric pressure, with, besides the toluene used, the excess of allyl chloride (66 g) being recovered.

4-Allyloxy-2,2,6,6-tetramethylpiperidine, purified by distillation of the residue (b.p. 64-65 °C (1,333 hPa) (1 $mm_{Hg}$) was characterized by I.R., N.M.R. and mass spectroscopy. The reaction yield computed relatively to charged 2,2,6,6-tetramethylpiperidinol resulted of 73%, and the yield relative to allyl chloride, with the chloride recovered on distillation being considered recyclable, resulted of 88%.

Examples 2 to 6

Examples 2-6 were carried out as reported in Example 1, but using different phase-transfer catalysts, in the same amount, by mol, relatively to the substrate (piperidinol mol/catalyst mol = about 400). The experimental results are reported in the following Table.

| Example No. | Catalyst | Piperidinol % Conversion | Piperidinol % Selectivity | Allyl Chloride % Selectivity |
|---|---|---|---|---|
| 2 | Tetrabutylammonium Chloride (TBAC) | 93 | 98 | 87 |
| 3 | Tetrabutylammonium Bromide (TBAB) | 97 | 97 | 90 |
| 4 | Tetrapropylammonium Bromide (TPAB) | 95 | 97 | 88 |
| 5 | Hexadecyltrimethylammonium Fluoride (ETAF) | 85 | 91 | 75 |
| 6 | Crown Ether 18.6 | 73 | 90 | 81 |

Example 7 to 9

The following Examples were carried out by substantially following the method as disclosed in Example 1, and using the compounds listed in the following Table, in the "Substrate" headed column, instead of

2,2,6,6-tetramethylpiperidinol. The oatalyst and the amounts used are shown in the Table. The corresponding allylethers obtained were characterized by I.R., N.M.R. and mass spectroscopy.

| Example No. | Substrate | Catalyst | Substrate Mol / Catalyst Mol | % Yield Relatively to the Substrate | Allyl Chloride % Selectivity | Product B.p. (°C) |
|---|---|---|---|---|---|---|
| 7 | 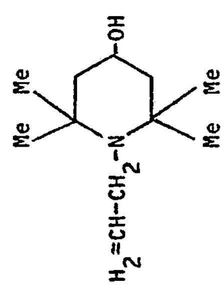 | TBAB | 500 | 92 | 94 | 92-3 (0.4 $mm_{Hg}$) |
| 8 | 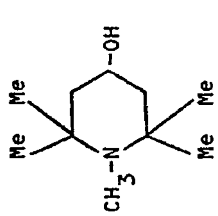 | TBAB | 400 | 93 | 93 | 116-8 (12 $mm_{Hg}$) |
| 9 | 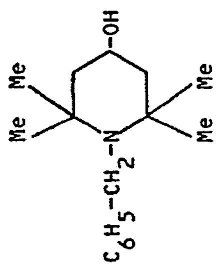 | TBAB | 200 | 83 | 87 | 115-20 (1 $mm_{Hg}$) (1.333 hPa) |

Examples 10 to 17

The following Examples report the influence of the base nature and amount on the yield, as referred to 2,2,6,6-tetramethylpiperidinol. The reactions were carried out according to as reported in Example 1, by using TBAB as the catalyst (substrate mol/catalyst mol = 400). The reaction time is 6 hours.

| No. | Base | Substrate Mol/ Base Mol | Yield Relatively to the Substrate |
|---|---|---|---|
| 10 | NaOH | 0.3 | 95 |
| 11 | NaOH | 0.4 | 91 |
| 12 | NaOH | 0.54 | 93 |
| 13 | NaOH | 0.6 | 93 |
| 14* | NaOH | 0.8 | 82 |
| 15 | $K_2CO_3$ | 0.6 | 10 |
| 16 | $NaHCO_3$ | 0.6 | 10 |
| 17 | $Ca(OH)_2$ | 0.6 | 10 |

* By prolonging the reaction times (beyond 12 hours), also in this case a yield higher than 90% is obtained.

Examples 18-20

In the following Table the results are reported, which were obtained by carrying out the reaction substantially as disclosed in Example 1, but using TBAB as the catalyst, and varying the molar ratio thereof relatively to the substrate. The yields of allyloxypiperidine were measured after a 3-hour reaction time at refluxing temperature.

| No. | Piperidinol Mol/ Catalyst Mol | Yield % |
|---|---|---|
| 18 | 160 | 99.5 |
| 19 | 360 | 89 |
| 20 | 1075 | 56 |

Example 21 to 23

The reactions of the following examples were carried out by operating according to the known art of the phase transfer, by using a concentrated aqueous solution of NaOH. The reactions were carried out with 2,2,6,6-tetramethylpiperidinol as the substrate, and TBAB as the catalyst (piperidinol mol/TBAB mol = 50).

| Example No. | Substrate Weight / $H_2O$ Weight | Substrate Weight / NaOH Weight | Piperidinol % Yield | Allyl Chloride % Selectivity | Reaction Time (hours) |
|---|---|---|---|---|---|
| 21 | 0.5 | 0.5 | 94 | 70 | 2 |
| 22 | 1 | 1 | 95 | 71 | 3 |
| 23 | 0.5 | 1 | 86 | 68 | 3 |

Example 24

Preparation of 4-[(2-methyl-2-propenyl)-oxy]-2,2,6,6-tetramethylpiperidine

Substantially following the same procedure as of Example 1, but using 3-chloro-2-methylpropene (119 g, 1.3 mol) instead of allyl chloride, the compound of the title is obtained with a yield of 92% relatively to charged piperidinol, and a selectivity higher than 98% (b.p. 50 °C/(0,2666 hPa) 0.2 $mm_{Hg}$).

## Claims

1. Process for the synthesis of a 4-allyloxy-2,2,6,6-tetraalkylpiperidinic derivative having the formula (I):

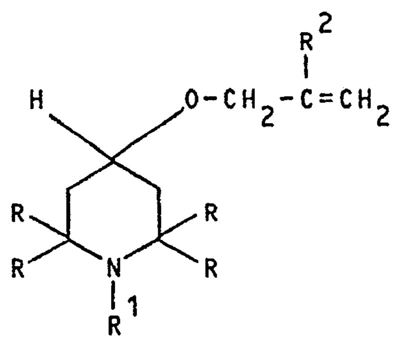

(I)

wherein the four substituents R are alkyl groups containing from 1 to 4 carbon atoms, $R^1$ is a hydrogen atom, an alkyl group containing from 1 to 20 carbon atoms, an alkenyl group containing from 3 to 20 carbon atoms, a phenylalkyl group containing from 7 to 12 carbon atoms, or a group having the formula

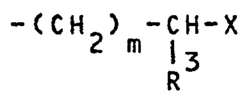

wherein m is 0, 1, 2, or 3, $R^3$ is hydrogen, methyl or phenyl and X represents a halogen atom or a cyano, -COR$^4$, -COOR$^4$, -COSR$^4$, -CONR$^4$R$^5$, or -CSNR$^4$R$^5$ group, wherein $R^4$ is an alkyl group containing from 1 to 4 carbon atoms, and $R^5$ is a hydrogen atom or an alkyl group containing from 1 to 4 carbon atoms, and $R^2$ is either hydrogen or a methyl group, by means of the allylation of the corresponding piperidinol having the formula (II):

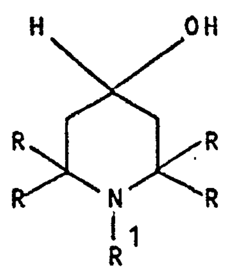

(II)

wherein R and $R^1$ have the above-defined meanings, with an allyl halide of formula (III)

$CH_2 = CR^2\text{-}CH_2Y$ (III)

wherein $R^2$ is as defined above, and Y is a chlorine or bromine atoms, characterized in that the piperidinic derivative of formula (II) is contacted with an at least equimolar amount of the allyl halide in the presence of an at least equimolar amount of a finely subdivided alkaline hydroxide and of catalytic amounts of a phase-transfer catalyst, in the absence of solvents, or in the presence of an inert organic solvent

2. Process according to claim 1, wherein the allyl halide is used in a large excess.

3. Process according to claim 2, wherein such excess is comprised within the range of from 100 to 400% by mol, relatively to the stoichiometric amount.

4. Process according to claim 1, wherein an excess is used of alkaline hydroxide.

5. Process according to claim 4, wherein such excess is comprised within the range of from 30% to 150% by mol relatively to the stoichiometric amount.

6. Process according to claim 1, wherein the phase-transfer catalyst is selected from quaternary ammonium salts.

7. Process according to claim 1, wherein the phasetransfer catalyst is used in an amount comprised within the range of from 0.005 to 2% by mol relatively to the starting compound of formula (II).

8. Process according to claim 7, wherein the phasetransfer catalyst is used in an amount comprised within the range of from 0.1% to 0.5% by mol relatively to the compound of formula (II) used as the starting compound.

9. Process according to claim 1, wherein no solvents are used.

10. Process according to claim 1, wherein the inert organic solvent is selected from the alkyl ethers and the cyclic ethers.

11. Process according to claim 1, wherein the alkaline hydroxide is selected from sodium hydroxide and potassium hydroxide.

12. Process according to claim 1, wherein the temperature is comprised within the range of from 20 to 120 °C.

13. Process according to claim 12, wherein the reaction is carried out at the reflux temperature of the reaction mixture.

14. Process according to any of preceding claims, for preparing a compound of formula (I), wherein R is methyl, $R^1$ is a hydrogen atom, an alkyl group containing from 1 to 20 carbon atoms, an alkenyl group containing from 3 to 20 carbon atoms, or a phenylalkyl group containing from 7 to 12 carbon atoms, and $R^2$ is either hydrogen or methyl.

15. Process according to claim 14, wherein $R^1$ is a hydrogen atom, an alkyl group containing from 1 to 20 carbon atoms, or an alkenyl group containing from 3 to 20 carbon atoms.

## Revendications

1. Procédé de synthèse d'un dérivé 4-allyloxy-2,2,6,6-tétraalkylpipéridinique de formule (I) :

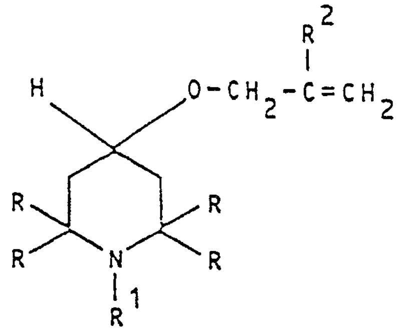

(I)

dans laquelle les quatre substituants R représentent des groupes alkyle ayant de 1 à 4 atomes de carbone, $R^1$ représente un atome d'hydrogène, un groupe alkyle ayant de 1 à 20 atomes de carbone,

un groupe alcényle ayant de 3 à 20 atomes de carbone, un groupe phénylalkyle ayant de 7 à 12 atomes de carbone ou un groupe de formule :

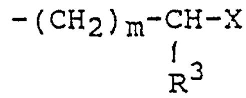

dans laquelle m est 0, 1, 2 ou 3, $R^3$ est un atome d'hydrogène, un groupe méthyle ou phényle et X représente un atome d'halogène ou un groupe cyano, $-COR^4$, $-COOR^4$, $-COSR^4$, $-CONR^4R^5$ ou $-CSNR^4R^5$, groupes dans lesquels $R^4$ est un groupe alkyle ayant de 1 à 4 atomes de carbone et $R^5$ est un atome d'hydrogène ou un groupe alkyle ayant de 1 à 4 atomes de carbone, et $R^2$ est un atome d'hydrogène ou un groupe méthyle, par allylation du pipéridinol correspondant de formule (II) :

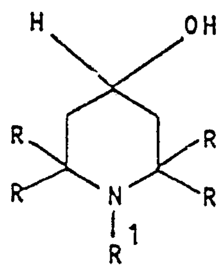

(II)

dans laquelle R et $R^1$ ont les significations indiquées précédemment, au moyen d'un halogénure d'allyle de formule (III) :

$CH_2 = CR^2\text{-}CH_2Y$ (III)

dans laquelle $R^2$ est tel que défini précédemment et Y est un atome de chlore ou de brome, **caractérisé** en ce que l'on met en contact le dérivé pipéridinique de formule (II) avec une quantité au moins équimolaire de l'halogénure d'allyle, en présence d'une quantité au moins équimolaire d'un hydroxyde alcalin finement divisé et de quantités catalytiques d'un catalyseur de transfert de phase, en l'absence de solvant ou en présence d'un solvant organique inerte.

**2.** Procédé selon la revendication 1, dans lequel on utilise un grand excès de l'halogénure d'allyle.

**3.** Procédé selon la revendication 2, dans lequel ledit excès représente de 100 à 400 % en moles de la quantité stoechiométrique.

**4.** Procédé selon la revendication 1, dans lequel on utilise un excès d'hydroxyde alcalin.

**5.** Procédé selon la revendication 4, dans lequel ledit excès représente de 30 % à 150 % en moles de la quantité stoechiométrique.

**6.** Procédé selon la revendication 1, dans lequel le catalyseur de transfert de phase est choisi parmi les sels d'ammonium quaternaire.

**7.** Procédé selon la revendication 1, dans lequel on utilise le catalyseur de transfert de phase en une proportion comprise dans l'intervalle allant de 0,005 à 2 % en moles par rapport au composé de départ de formule (II).

**8.** Procédé selon la revendication 7, dans lequel on utilise le catalyseur de transfert de phase en une proportion comprise dans l'intervalle allant de 0,1 % à 0,5 % en moles par rapport au composé de formule (II) utilisé comme composé de départ.

**9.** Procédé selon la revendication 1, dans lequel on n'utilise pas de solvant.

**10.** Procédé selon la revendication 1, dans lequel le solvant organique inerte est choisi parmi les éthers alkyliques et les éthers cycliques.

**11.** Procédé selon la revendication 1, dans lequel l'hydroxyde alcalin est choisi parmi l'hydroxyde de sodium et l'hydroxyde de potassium.

**12.** Procédé selon la revendication 1, dans lequel la température est comprise dans l'intervalle allant de 20 à 120 °C.

**13.** Procédé selon la revendication 12, dans lequel on effectue la réaction à la température de reflux du mélange réactionnel.

**14.** Procédé selon l'une quelconque des revendications précédentes, pour la préparation d'un composé de formule (I), dans laquelle R est un groupe méthyle, $R^1$ est un atome d'hydrogène, un groupe alkyle ayant de 1 à 20 atomes de carbone, un groupe alcényle ayant de 3 à 20 atomes de carbone ou un groupe phénylalkyle ayant de 7 à 12 atomes de carbone et $R^2$ est un atome d'hydrogène ou un groupe méthyle.

**15.** Procédé selon la revendication 14, dans lequel $R^1$ est un atome d'hydrogène, un groupe alkyle ayant de 1 à 20 atomes de carbone ou un groupe alcényle ayant 3 à 20 atomes de carbone.

## Ansprüche

**1.** Verfahren zur Herstellung eines 4-Allyloxy-2,2,6,6-tetraalkylpiperidinderivats mit der Formel (I):

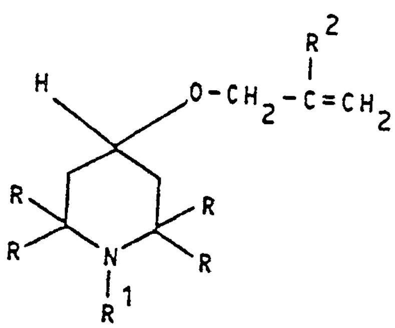

(I),

worin die vier Substituenten R Alkylgruppen mit 1 bis 4 Kohlenstoffatomen bedeuten, $R^1$ ein Wasserstoffatom, eine Alkylgruppe mit 1 bis 20 Kohlenstoffatomen, eine Alkenylgruppe mit 3 bis 20 Kohlenstoffatomen, eine Phenylalkylgruppe mit 7 bis 12 Kohlenstoffatomen oder eine Gruppe mit der Formel:

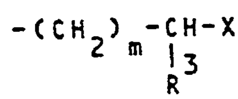

bedeutet, worin m den Wert 0, 1, 2 oder 3 aufweist, $R^3$ für Wasserstoff, Methyl oder Phenyl steht und X ein Halogenatom oder eine Cyanogruppe, $-COR^4$-Gruppe, $-COOR^4$-Gruppe, $-COSR^4$-Gruppe, $-CONR^4R^5$-Gruppe oder $-CSNR^4R^5$-Gruppe darstellt, worin $R^4$ eine 1 bis 4 Kohlenstoffatome enthaltende Alkylgruppe bedeutet und $R^5$ ein Wasserstoffatom oder eine Alkylgruppe mit 1 bis 4 Kohlenstoffatomen darstellt, und $R^2$ entweder Wasserstoff oder eine Methylgruppe bedeutet, durch Allylierung des entsprechenden Piperidinols mit der Formel (II):

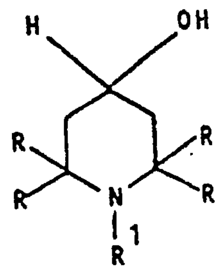

(II)

worin R und $R^1$ die vorstehend definierten Bedeutungen aufweisen, mit einem Allylhalogenid der Formel (III):

$CH_2 = CR^2\text{-}CH_2Y$ (III),

worin $R^2$ wie vorstehend definiert ist und Y ein Chlor- oder Bromatom bedeutet, dadurch gekennzeichnet, daß das Piperidinderivat der Formel (II) mit einer wenigstens äquimolaren Menge des Allylhalogenids in Gegenwart einer wenigstens äquimolaren Menge eines feinverteilten Alkalihydroxids und von katalytischen Mengen eines Phasentrans ferkatalysators in Abwesenheit von Lösungsmitteln oder in Anwesenheit eines inerten organischen Lösungsmittels in Berührung gebracht wird.

2. Verfahren nach Anspruch 1, worin das Allylhalogenid in einem großen Überschuß eingesetzt wird.

3. Verfahren nach Anspruch 2, worin der Überschuß im Bereich von 100 bis 400 Mol-%, bezogen auf die stöchiometrische Menge, liegt.

4. Verfahren nach Anspruch 1, worin ein Überschuß an Alkalihydroxid eingesetzt wird.

5. Verfahren nach Anspruch 4, worin der Überschuß im Bereich von 30 bis 150 Mol-%, bezogen auf die stöchiometrische Menge, liegt.

6. Verfahren nach Anspruch 1, worin der Phasentransferkatalysator unter quaternären Ammoniumsalzen ausgewählt wird.

7. Verfahren nach Anspruch 1, worin der Phasentransferkatalysator in einer Menge im Bereich von 0,005 bis 2 Mol-%, bezogen auf die eingesetzte Verbindung der Formel (II), verwendet wird.

8. Verfahren nach Anspruch 7, worin der Phasentransferkatalysator in einer Menge im Bereich von 0,1 bis 0,5 Mol-%, bezogen auf die als Ausgangsverbindung verwendete Verbindung der Formel (II), verwendet wird.

9. Verfahren nach Anspruch 1, worin keine Lösungsmittel eingesetzt werden.

10. Verfahren nach Anspruch 1, worin das inerte organische Lösungsmittel unter den Alkylethern und den cyklischen Ethern ausgewählt wird.

11. Verfahren nach Anspruch 1, worin das Alkalihydroxid unter Natrium-hydroxid und Kaliumhydroxid ausgewählt wird.

12. Verfahren nach Anspruch 1, worin die Temperatur im Bereich von 20 bis 120° C gehalten wird.

13. Verfahren nach Anspruch 12, worin die Umsetzung bei der Rückfluß-temperatur des Reaktionsgemisches ausgeführt wird.

14. Verfahren nach einem der vorstehenden Ansprüche, zur Herstellung einer Verbindung der Formel (I), worin R Methyl bedeutet, $R^1$ ein Wasserstoffatom, eine Alkylgruppe mit 1 bis 20 Kohlenstoffatomen, eine Alkenylgruppe mit 3 bis 20 Kohlenstoffatomen oder eine Phenylalkylgruppe mit 7 bis 12 Kohlenstoffatomen bedeutet, und $R^2$ entweder Wasserstoff oder Methyl darstellt.

**15.** Verfahren nach Anspruch 14, worin $R^1$ ein Wasserstoffatom, eine Alkyl-gruppe mit 1 bis 20 Kohlenstoffatomen oder eine Alkenylgruppe mit 3 bis 20 Kohlenstoffatomen bedeutet.